Europäisches Patentamt

**European Patent Office**

Office européen des brevets

(11) Publication number: **0 097 440**
**B1**

(12) **EUROPEAN PATENT SPECIFICATION**

(45) Date of publication of patent specification: **24.09.86**

(51) Int. Cl.⁴: **G 01 N 33/537**

(21) Application number: **83303142.0**

(22) Date of filing: **01.06.83**

(54) Method and kit for removing and assaying complement system fragments.

(30) Priority: **14.06.82 US 388068**

(43) Date of publication of application:
**04.01.84 Bulletin 84/01**

(45) Publication of the grant of the patent:
**24.09.86 Bulletin 86/39**

(84) Designated Contracting States:
**BE CH DE FR GB IT LI NL SE**

(56) References cited:
**EP-A-0 009 198**

**CHEMICAL ABSTRACTS, vol. 87, no. 17, 24th October 1977, page 488, no. 132023r, Columbus, Ohio, USA K. MIYATANI et al.: "Serum fractionation with rivanol solution and its ASO antibody titer"**

**CHEMICAL ABSTRACTS, vol. 94, no. 17, 27th April 1981, page 576, no. 137515g, Columbus, Ohio, USA T. E. HUGLI et al.: "Biologically active peptides of complement: techniques and significance of C3a and D5a measurements"**

(73) Proprietor: **THE UPJOHN COMPANY**
**301 Henrietta Street**
**Kalamazoo, Michigan 49001 (US)**

(72) Inventor: **Satoh, Paul Shigemi**
**c/o The Upjohn Company 301 Henrietta Street**
**Kalamazoo Michigan 49001 (US)**

(74) Representative: **Perry, Robert Edward et al**
**GILL JENNINGS & EVERY 53-64 Chancery Lane**
**London WC2A 1HN (GB)**

(56) References cited:

**CHEMICAL ABSTRACTS, vol. 86, no. 17, 25th April 1977, page 216, no. 117179g, Columbus, Ohio, USA M. GIBOWSKI et al.: "Elimination of nonspecific streptolysin O inhibitors by rivanol"**

Courier Press, Leamington Spa, England.

## Description

The present invention relates to a method for assaying complement fragments $C_3a$, $C_4a$ and $C_5a$ or the des-Arg derivatives thereof, and a mercantile kit useful in performing said immunoassay.

The complement system of humans and other mammals involves more than 20 components which participate in an orderly sequence of reactions resulting in complement activation. Numerous studies indicate that the complement system is a fundamental element of normal host defence mechanisms. As a consequence, complement activation is commonly associated with a variety of pathological states such as certain malignancies, myocardial infarction, systemic lupus erythematosus, and adult respiratory distress syndrome. Because of these correlations clinical laboratory methods that detect complement activation are useful in diagnosing certain disease conditions.

Complement activation can occur by either of two primary modes known as the "classical" pathway and the "alternative" pathway, respectively. These different pathways are generally distinguished according to the process which initiates complement activation. Activation via the classical pathway is usually associated with an immunologic stimulus whereas activation via the alternative pathway is most commonly associated with non-immunologic stimuli. Regardless of the initiating stimulus both pathways converge, followed by the conversion of the $C_3$ component of complement into its $C_3a$ and $C_3b$ fragments. This cleavage of $C_3$ into its sub-components is considered to be one of the significant events signalling activation of the alternative complement cascade. Following the conversion of $C_3$, a $C_5$ convertase enzyme complex is formed. This enzyme cleaves the $C_5$ component to yield the fragments $C_5a$ and $C_5b$. Complement activation by the classical pathway mechanism is uniquely characterized by the fact that this route leads to the conversion of the $C_4$ component to its fragments $C_4a$ and $C_4b$.

The physicochemical and physiological properties of the cleavage products $C_3a$, $C_4a$ and $C_5a$, termed anaphylatoxins, are well known. Each is a potent bioactive polypeptide and plays a key role as a mediator of acute inflammatory processes. Among these three anaphylatoxins $C_5a$ alone is uniquely characterized by its ability to interact with white blood cells. Both $C_3a$ and $C_4a$ are rendered inactive in vivo by conversion to their respective des-arginine derivatives ($C_3a_{des-Arg}$ or $C_3a_i$, $C_4a_{des-Arg}$ or $C_4a_i$) by a serum carboxypeptidase. Human $C_5a$, on the other hand, is converted to $C_5a_{des-Arg}$ by this serum carboxypeptidase only after all available white blood cell binding sites for $C_5a$ have been saturated.

Conversion of the human complement components $C_3$ and $C_5$ to yield their respective anaphylatoxin products has been implicated in certain naturally occurring pathologic states including: autoimmune disorders such as systemic lupus erythematosus, rheumatoid arthritis, malignancy, myocardial infarction, Purtscher's retinopathy, and adult respiratory distress syndrome. In addition, increased circulating levels of $C_3a$ and $C_5a$ have been detected in certain conditions associated with iatrogenic complement activation such as: cardiopulmonary bypass surgery, renal dialysis, and nylon fiber leukaphoresis. Elevated levels of $C_4a$ anaphylatoxin are commonly associated with the autoimmune disorders mentioned above. Therefore, the ability to quantitatively measure the circulating levels of these anaphylatoxins or their des-Arg derivatives is of great utility in diagnosing a variety of important pathological conditions. Additionally, the ability to measure levels of $C_4a$ or $C_4a_{des\ Arg}$ enables one to determine the pathway by which complement activation occurs. This facility enables one not only to determine the precise mechanism of complement activation but also whether a patient's natural immunological defence mechanisms are functional.

Until the development of the radioimmunoassay (RIA) method of Tony E. Hugli and Dennis E. Chenoweth reported in "Immunoassays: Clinical Laboratory Techniques for the 1980s", 443—460, Alan R. Liss, Inc., New York, NY (1980), measurement of the anaphylatoxins $C_3a$, $C_4a$ and $C_5a$ or their des-Arg derivatives had only been achieved when the levels of these factors were relatively elevated, for example, when the disease process had reached an advanced stage. The RIA techniques of Hugli and Chenoweth permit quantitative measurement of trace amounts of the anaphylatoxins or their des-Arg derivatives and hence provide a sensitive diagnostic tool. However, the means known heretofore for measuring these factors have been fraught with a significant problem associated with the requirement that the $C_3$, $C_4$ and $C_5$ plasma precursors of the anaphylatoxins must be removed from the biological fluid to be tested. This stringent requirement is predicated on the observation that the antibodies raised to the anaphylatoxins possess a significant cross-reactivity with their respective plasma precursor, as $C_3a$, $C_4a$ or $C_5a$ is a part of the parent molecule $C_3$, $C_4$ or $C_5$, respectively. Because of this unavoidable cross-reactivity, it is imperative that the precursor (which exists in relatively high concentrations in serum and plasma) be completely removed, to avoid detecting artifactually elevated levels of the anaphylatoxins (which are normally present in only trace amounts). Known methods of separating the anaphylatoxins from their plasma precursors involve diluting the plasma with sodium chloride, acidifying with hydrochloric acid, and subsequently neutralising the recovered serum sample; see Hugli and Chenoweth, *supra*.

C.A. *87* 132023r discloses the use of 2-ethoxy-6,9-acridinediamine lactate monohydrate, also known as acrinol and rivanol, for serum fractionation.

According to the present invention, a method for assaying a complement fragment selected

from $C_3a$, $C_4a$, $C_5a$ and the des-Arg derivatives thereof, in a biological sample, comprises incubating a mixture of equal volumes of the biological sample and a 0.8 to 1.6% solution of a reagent which is 2-ethoxy-6,9-acridinediamine or the lactate monohydrate salt thereof for from one minute to 2 hours, whereby a precipitate is formed, recovering the supernatant, incubating the supernatant with a known amount of the labelled complement fragment and a known amount of antibody which recognises the complement fragment, separating the free labelled complement fragment from the bound labelled complement fragment, measuring the amount of labelled complement fragment in either the free or antibody-bound complement component, and determining the concentration of complement fragment in the biological sample by comparison to a standard curve.

According to a further aspect of the present invention, a method for removing a complement component selected from $C_3$, $C_4$ and $C_5$ from a sample of a biological fluid, and recovering from the fluids a complement fragment as defined above, comprises combining equal volumes of the biological fluid sample and a buffered solution of 0.8 to 1.6% of a reagent as defined above, incubating the mixture for from one minute to 2 hours at about 25°C, whereby a precipitate is formed, and recovering the supernatant containing the complement fragment.

According to a third aspect of the invention, a mercantile kit, of which the components can be assembled for use in assaying biological fluids for a complement fragment as defined above comprises.

(a) a first container having therein a buffered solution of 0.8 to 1.6% of a reagent as defined above;

(b) a second container having therein the labelled complement fragment; and

(c) a third container having therein an antibody which recognises the complement fragment.

A kit according to the invention preferably additionally comprises

(a) a fourth container having therein assay buffer;

(b) a fifth container having therein a second antibody;

(c) a sixth container having therein the complement fragment standard, 25 ng;

(d) a seventh container having therein the complement fragment standard, 10 ng;

(e) an eighth container having therein the complement fragment standard, 5.0 ng;

(f) a ninth container having therein the complement fragment standard, 2.5 ng; and

(g) a tenth container having therein the complement fragment standard, 1.0 ng.

A kit of the invention comprises multiple containers such as bottles. The antibody in the third container is preferably rabbit antiserum to the particular complement fragment. The second antibody, in the fifth container (if present), binds the product of the initial complement fragment-antibody reaction; it is preferably goat anti-rabbit serum.

The present invention provides a novel and simplified means of quantitatively removing the plasma precursor of the anaphylatoxin from the biological samples, yet simultaneously permitting quantitative recovery of the low molecular weight anaphylatoxins, $C_3a$, $C_4a$, $C_5a$ and the des-Arg derivatives thereof. Also, the present invention provides a novel method for removing substantially all traces of complement precursors or components $C_3$, $C_4$ and $C_5$ from samples of biological fluids and recovering complement fragments without interfering with the immunogenicity of the fragments.

The preferred reagent is acrinol. The invention will now be described by way of example with reference to the use of acrinol, although 2-ethoxy-6,9-acridinediamine can be used instead.

In practising the method of the present invention, the sample of biological fluid can be any body fluid such as, for example, serum, plasma, urine or cerebrospinal fluid. When the biological fluid is plasma or cerebrospinal fluid, EDTA is added thereto.

Equal volumes of the biological fluid and 0.8 to 1.6% acrinol are combined, for a final concentration of 0.4 to 0.8% acrinol. The preferred final concentration of acrinol is 0.4%. The acrinol protein complex is buffered to a pH of about 7.4. Any buffer which does not contain chloride ions is suitable, and examples are carbonate buffers, phosphate buffers and HEPES [2 - (N - 2 - hydroxyethyl - N' - piperazinyl) - ethanesulfonic acid]. Preferably, the molarity of the buffer is between 0.05 to 0.1M, with a pH of from 6.8 to 7.8. The preferred buffer is 0.05M phosphate buffer, pH 7.4.

Once the biological fluid and acrinol are combined, a precipitate forms almost immediately. However, the complex is preferably permitted to incubate for at least 20 minutes, to effect maximum separation of the complement precursors $C_3$, $C_4$ and $C_5$ from their activated fragments. It has been found that precipitation of the complement component is complete within one minute to 2 hours. The complex may be permitted to incubate longer than 2 hours, if desired, but no beneficial additional separation of protein is expected to occur beyond 2 hours. Incubation is carried out at room temperature, that is, about 25°C.

Removal of the precipitate is conveniently accomplished by centrifuging the acrinol protein complex at about 3,000 to 7,000×g for about 10 to 20 minutes then decanting the supernatant which contains the complement fragments $C_3a$, $C_4a$ and $C_5a$ or the des-Arg derivatives thereof. As indicated hereinabove, complement fragments $C_3a$, $C_4a$ and $C_5a$ are rendered inactive in vivo in serum and plasma by carboxypeptidases by conversion to their respective inactive des-Arg derivatives. Such conversion also occurs in vivo in urine. Hence, the supernatant recovered from the novel acrinol precipitation method of the present invention will contain primarily the des-Arg derivative of the complement fragments $C_3a$, $C_4a$ and $C_5a$ rather than the activated form of said fragments when the biological fluid is plasma, serum or urine. For diagnostic purposes measurement of the inactive des-Arg derivative is just as meaningful as the direct measurement of the active form of the complement fragments.

The recovered supernatant is used without further treatment or processing in the immunoassay procedure. We have found that the presence of acrinol in the test samples does not interfere with the antigenicity of the complement fragments or the des-Arg derivatives thereof, thus providing a unique and greatly simplified means of assaying for said fragments.

The immunoassay procedure is not substantially different from known immunoassays for $C_3a$, $C_4a$ and $C_5a$ or the des-Arg derivatives thereof. Equal volumes of the test sample to be assayed, labeled complement fragment $C_3a$, $C_4a$ or $C_5a$ or the des-Arg derivative thereof, and antibody which recognizes said complement fragment are combined and incubated after which the antibody bound and unbound, or free, labeled complement fragment are separated and measured to determine the amount of complement fragment in the test sample by comparison to standard curves. An assay buffer is generally added to the incubate. We have found a buffer comprising HEPES, protamine sulfate, thimerosal and gelatin to be particularly useful.

Purification of the complement fragment $C_3a$, $C_4a$ or $C_5a$ or the des-Arg derivative thereof for use in raising antibody thereto, and for use in generating standard curves as well as for preparing labeled complement fragment can be achieved by the general method described in J. Biol. Chem. 256, 8685—8692 (1981). We have found that this procedure can be modified and improved by substituting the acrinol precipitation technique described hereinabove for the hydrochloric acid precipitation technique.

Antibody to complement fragments $C_3a$, $C_4a$, $C_5a$ or the des-Arg derivatives thereof is raised as generally described by Hugli, et al., J. Biol. Chem. 250, 1472—1478 (1975), and in J. Biol. Chem. 256, 8685—8692 (1981).

The label utilized in the labeled complement fragment or the des-Arg derivative thereof can be any substance capable of detection by physical or chemical means. Radioisotopes such as tritium iodine-131 and iodine-125 are useful with [125]I being preferred. The [125]I labeled material can be prepared by various means generally known in the art, such as, the solid-phase lactoperoxidase method. A preferred method employs the use of TCDG (1,3,4,6 - tetrachloro - 3α,6α - diphenylglycouril) in phosphate buffered saline.

The antibody bound complement fragment can be separated from the free or unbound complement fragment by various means commonly employed in immunoassay procedures. For example, this separation can be achieved by treatment with polyethylene glycol [Desbuquois, B. and Aurback, G. D., J. Clin. Endocrinol. Metab. 33, 732 (1971)] or IgG Sorb or by contacting the incubate with a second antibody. The second antibody, which is prepared by standard procedures, for example, as described in Daughaday, et al., "Principle of Competitive Protein Binding Assay", J. B. Lippincott, Philadelphia (1971), recognizes the complement fragment-antibody complex contained in the incubate. Use of the second antibody technique is particularly preferred.

Standard curves are derived essentially by performing the above-described assay procedure using known quantities of complement fragment $C_3a$, $C_4a$ or $C_5a$ or the des-Arg derivative thereof in place of the test sample. The assay procedure described herein is highly sensitive, and we have found it particularly useful to develop standard curves for concentrations of the complement fragments ranging from 1.0 ng to 25 ng.

The following examples further illustrate the invention.

Example 1
Purification of complement fragments or the des-Arg derivative

From 2 to 4 liters of serum were activated at 37°C by addition of boiled yeast (20 mg/ml serum) and allowing the mixture to stand for 45 minutes to one hour. Equal volumes of activated serum and 0.8 to 1.6% acrinol, buffered to pH 7.4 with 0.05M phosphate buffer, were combined and let stand for 30 minutes at 25°C after which the mixture was centrifuged and the supernatant was decanted. The supernatant was dialyzed against running water overnight at 5°—8°C then gel filtered on a p-60 column equilibrated with 0.1M ammonium formate, pH 5.0. The fractions containing complement fragments $C_3a$, $C_4a$ or $C_5a$ or the des-Arg derivatives thereof were pooled and applied to an SP-Sephadex® column equilibrated with 0.1M ammonium formate, pH 5.0. The column was eluted by a linear gradient of 0.1M to 0.8M ammonium formate, pH 7.0. The fractions of $C_3a$, $C_4a$ and $C_5a$ or the des-Arg derivatives thereof were collected and applied individually to separate CM-cellulose columns and eluted by a gradient of 0.15M to 0.43M ammonium formate, pH 7.0, at a flow rate of 75 ml/hour. Each of the complement components $C_3a$, $C_4a$ or $C_5a$ was pooled with its respective des-Arg derivative recovered from the column and lyophilized then redissolved in water and dialyzed against 1% acetic acid. A final lyophilization and resuspension in water of about 10 mg/ml provided each fragment ready for use.

Example 2
[125]I Complement $C_3a$ or the des-Arg derivative thereof

To an incubation tube were added 50 μg of complement fragment $C_3a$ or the des-Arg derivative thereof, 150 μl of phosphate buffered saline, 100 μg of TCDG and 1mCi (10 μl) sodium iodide

($^{125}$INa). The phosphate buffered saline was prepared as a stock solution comprising 81 mg sodium chloride, 39 mg anhydrous sodium biphosphate, 199 mg sodium phosphate dibasic heptahydrate reagent and 100 ml glass distilled dionized water. The mixture was incubated for 20 minutes at about 25°C then transferred to a chromatography column equilibrated with phosphate buffered saline with gelatin which was prepared as a stock solution comprising 143 g sodium chloride, 200 mg thimerosal NF, 6 g anhydrous sodium biphosphate, 34 g sodium phosphate dibasic heptahydrate reagent, 17 g gelatin and glass distilled deionized water to a volume of 17.5 liters. Radioactive fractions of 0.5 ml each were collected and diluted to give 20,000 to 60,000 cpm/0.05 ml in phosphate buffered saline with gelatin.

Example 3

(a) A mixture of 0.45 ml activated plasma, 0.45 ml of 0.8% acrinol in 0.05M phosphate buffer, pH 7.4, and 0.1 ml distilled water was reacted at about 25°C for 20 minutes then centrifuged at 1700×g for 10 minutes. The supernatant was collected. A mixture of 50 µl of assay buffer prepared as described hereinabove, 50 µl of supernatant obtained above, 50 µl of $^{125}$I complement fragment $C_3a$ or the des-Arg derivative thereof and 50 µl of complement fragment $C_3a$ rabbit antisera was incubated for 30 minutes at about 25°C after which 50 µl of goat anti-rabbit antisera was added and mixed well. The mixture was incubated for an additional 30 minutes at about 25°C after which 2 ml of isotonic saline were added. The mixture was centrifuged at 2000 g for 10 minutes and the supernatant decanted. The radioactivity of the pellet was 4555 cpm.

(b) The foregoing procedure was performed only the acid-precipitation technique of Hugli and Chenoweth described in Immunoassays: Clinical Laboratory Techniques for the 1980s: 443—460 was substituted for the acrinol precipitation step. The radioactivity of the resultant pellet was 4521—4757 cpm demonstrating that the acrinol precipitation technique removes intact complement protein precursor as effectively as treatment with hydrochloric acid followed by base neutralization providing a greatly improved simplified assay procedure. Also the results of the two experiments indicate that the presence of acrinol does not affect either the first or the second immunological reactions.

In generating standard curves for complement fragment $C_3a$ or the des-Arg derivative thereof using 1.0 ng, 2.5 ng, 5.0 ng, 10 ng, 25 ng and 50 ng quantities of $C_3a$ we found that use of the acrinol precipitation step in place of the acid precipitation-base neutralization step of Hugli and Chenoweth increased the sensitivity of the immunoassay by a factor of 12 as evidenced by the fact that the range of detection became shorter and the slope %B/Bo (Standard Counts/ Bound Standard) was increased from −2.3 to −4.0.

Example 4

The assay as described in 3(a) above was performed using activated plasma samples to which known quantities of complement factor $C_3a$ or the des-Arg derivative thereof ranging from 2 to 20 ng were added. The results indicate that there were 100% of the known $C_3a$ concentrations in each of the "spiked" normal samples assayed.

**Claims**

1. A method for assaying a complement fragment selected from $C_3a$, $C_4a$, $C_5a$ and the des-Arg derivatives thereof, in a biological sample, which comprises incubating a mixture of the biological sample and a reagent, whereby a precipitate is formed, recovering the supernatant, incubating the supernatant with a known amount of the labelled complement fragment and a known amount of antibody which recognises the complement fragment, separating the free labelled complement fragment from the bound labelled complement fragment, measuring the amount of labelled complement fragment in either the free or antibody-bound complement component, and determining the concentration of complement fragment in the biological sample by comparison to a standard curve, characterised in that the reagent is 2-ethoxy-6,9-acridinediamine or the lactate monohydrate salt thereof (i.e. acrinol), that the admixture comprises equal volumes of the biological sample and a 0.8 to 1.6% solution of the reagent, and that the mixture is incubated for from 1 minute to 2 hours.

2. A method according to claim 1 wherein the reagent is acrinol and the solution thereof is buffered with 0.05M phosphate buffer, pH 7.4, and the label is $^{125}$I.

3. A method according to claim 2, wherein the free labelled complement fragment is separated from the bound labelled complement fragment by the addition of a second antibody.

4. A method for removing a complement component selected from $C_3$, $C_4$ and $C_5$ from a sample of a biological fluid, and recovering from the fluids a complement fragment as defined in claim 1, which comprises combining equal volumes of the biological fluid sample and a buffered solution of 0.8 to 1.6% of a reagent as defined in claim 1, incubating the mixture for from one minute to 2 hours at about 25°C, whereby a precipitate is formed, and recovering the supernatant containing the complement fragment.

5. A method according to claim 4, wherein the reagent is acrinol, the buffer is 0.05M phosphate buffer, pH 7.4, and the mixture is incubated for from 20 minutes to one hour.

6. A method according to any of claims 1 to 5, wherein the complement fragment is $C_3a$ or the des-Arg derivative thereof.

7. A method according to any of claims 1 to 5, wherein the complement fragment is $C_4a$ or the des-Arg derivative thereof.

8. A method according to any of claims 1 to 5, wherein the complement fragment is $C_5a$ or the des-Arg derivative thereof.

9. A mercantile kit, of which the components can be assembled for use in assaying biological fluids for a complement fragment as defined in claim 1, which comprises

(a) a first container having therein a buffered solution of 0.8 to 1.6% of a reagent as defined in claim 1;

(b) a second container having therein the labelled complement fragment; and

(c) a third container having therein an antibody which recognises the complement fragment.

10. A kit according to claim 9, wherein the reagent is acrinol, and which additionally comprises

(a) a fourth container having therein assay buffer;

(b) a fifth container having therein a second antibody;

(c) a sixth container having therein the complement fragment standard, 25 ng;

(d) a seventh container having therein the complement fragment standard, 10 ng;

(e) an eighth container having therein the complement fragment standard, 5.0 ng;

(f) a ninth container having therein the complement fragment standard, 2.5 ng; and

(g) a tenth container having therein the complement fragment standard, 1.0 ng.

## Patentansprüche

1. Eine Methode zur Untersuchung eines Komplementärfragments gewählt aus $C_3a$, $C_4a$, $C_5a$ und deren Des-Arg Derivaten in einer biologischen Probe, die die folgenden Vorgänge umfaßt: Inkubation einer Mischung der biologischen Probe und eines Reagenses, wodurch eine Ablagerung gebildet wird; Gewinnung der obenstehenden Flüssigkeit, Inkubation der obenstehenden Flüssigkeit mit einer bekannten Menge des markierten (indizierten) Komplementärfragments und einer bekannten Menge eines Antikörpers, der das Komplementärfragment erkennt, Trennung des freien markierten Komplementärfragments vom gebundenen markierten Komplementärfragment, Messung der Menge des markierten Komplementärfragments in der freien oder Antikörper-gebundenen Komplementärkomponente, und Bestimmung der Konzentration des Komplementärfragments in der biologischen Probe durch Vergleich mit einer Standardkurve, dadurch charakterisiert, daß das Reagens 2 - Ethoxy - 6,9 - Acridindiamin oder dessen Lactat - Monohydrat - Salz (d.h. Acrinol) ist, daß die Mischung gleiche Mengen der biologischen Probe und einer 0,8 bis 1,6% Lösung des Reagens enthält und daß die Mischung von 1 Minute bis zu 2 Stunden inkubiert wird.

2. Eine Methode gemäß Anspruch 1, wobei das Reagens Acrinol ist und dessen Lösung mit 0,05M Phosphatpuffer, pH 7.4, abgepuffert ist und die Markierung $^{125}I$ ist.

3. Eine Methode gemäß Anspruch 2, wobei das freie markierte Komplementärfragment durch das Hinzufügen eines zweiten Antikörpers von gebundenen markierten Komplementärfragment getrennt wird.

4. Eine Methode zur Entfernung einer Komplementärkomponente, gewählt aus $C_3$, $C_4$ und $C_5$, aus einer Probe einer biologischen Flüssigkeit und zur Gewinnung eines wie in Anspruch 1 definierten Komplementärfragments aus diesen Flüssigkeiten, bestehend aus der Kombination gleicher Mengen einer biologischen Flüssigkeitsprobe und einer gepufferten Lösung eines 0,8 bis 1,6% Reagenses wie in Anspruch 1 definiert, Inkubation der Mischung von 1 Minute bis zu 2 Stunden bei ungefähr 25°C, wodurch eine Ablagerung gebildet wird, und Gewinnung der obenstehenden, das Komplementärfragment enthaltenden Flüssigkeit.

5. Eine Methode gemäß Anspruch 4, wobei das Reagens Acrinol ist, der Puffer 0,05M Phosphatpuffer, pH 7,4, und die Mischung 20 Minuten bis zu 1 Stunde inkubiert wird.

6. Eine Methode gemäß einem der Ansprüche 1 bis 5, wobei das Komplementärfragment $C_3a$ oder dessen Des-Arg Derivat ist.

7. Eine Methode gemäß einem der Ansprüche 1 bis 5, wobei das Komplementärfragment $C_4a$ oder dessen Des-Arg Derivat ist.

8. Eine Methode gemäß einem der Ansprüche 1 bis 5, wobei das Komplementärfragment $C_5a$ oder dessen Des-Arg Derivat ist.

9. Ein Handelsausrüstung, deren Komponente zusammengestellt werden können zur Verwendung für die Untersuchung biologischer Flüssigkeiten auf ein wie in Anspruch 1 definiertes Komplementärfragment hin und die aus den folgenden Teilen besteht:

(a) einem ersten Behälter mit einer gepufferten Lösung von 0,8 bis 1,6% eines Reagenses wie in Anspruch 1 definiert ist;

(b) einem zweiten Behälter mit dem markierten Komplementärfragment; und

(c) einem dritten Behälter mit einem Antikörper, der das Komplementärfragment erkennt.

10. Eine Ausrüstung gemäß Anspruch 9, worin das Reagens Acrinol ist und welche darüberhinaus folgendes enthält:

(a) einen vierten Behälter mit Untersuchungspuffer;

(b) einen fünften Behälter mit einem zweiten Antikörper;

(c) einen sechsten Behälter mit dem Komplementärfragmentstandard, 25 ng;

(d) einen siebten Behälter mit dem Komplementärfragmentstandard, 10 ng;

(e) einen achten Behälter mit dem Komplementärfragmentstandard, 5,0 ng;

(f) einen neunten Behälter mit dem Komplementärfragmentstandard, 2,5 ng; und

(g) einen zehnten Behälter mit dem Komplementärfragmentstandard, 1,0 ng.

## Revendications

1. Méthode d'analyse d'un fragment de complé-

ment choisi entre $C_3a$, $C_4a$, $C_5a$ et leurs dérivés des-Arg dans un échantillon biologique, qui consiste à faire incuber un mélange de l'échantillon biologique et d'un réactif, de manière à former un précipité, à recueillir la liqueur surnageante, à faire incuber la liqueur surnageante avec une quantité connue du fragment de complément marqué et une quantité connue d'anticorps qui reconnaît le fragment de complément, à séparer le fragment de complément marqué libre du fragment de complément marqué lié, à mesurer la quantité de fragment de complément marqué dans le composant libre ou lié à l'anticorps du complément et à déterminer la concentration du fragment de complément dans l'échantillon biologique par comparaison avec une courbe de référence, caractérisée en ce que le réactif est la 2 - éthoxy - 6,9 - acridine - diamine ou son sel monohydraté d'acide lactique (c'est-à-dire l'acrinol), en ce que le mélange comprend des volumes égaux de l'échantillon biologique et d'une solution à 0,8—1,6% du réactif et en ce qu'on le fait incuber pendant une durée d'une minute à deux heures.

2. Méthode suivant la revendication 1, dans laquelle le réactif est l'acrinol et sa solution est tamponnée avec du tampon au phosphate 0,05M à pH 7,4 et l'indicateur est l'iode $^{125}$I.

3. Méthode suivant la revendication 2, dans laquelle le fragment de complément marqué libre est séparé du fragment de complément marqué lié, par l'addition d'un second anticorps.

4. Méthode pour éliminer un composant du complément choisi entre $C_3$, $C_4$ et $C_5$ d'un échantillon d'un liquide biologique et pour recueillir du liquide un fragment de complément tel que défini dans la revendication 1, qui consiste à réunir des volumes égaux de l'échantillon de liquide biologique et d'une solution tamponnée de 0,8 à 1,6% d'un réactif tel que défini dans la revendication 1, à faire incuber le mélange pendant une durée d'une minute à deux heures à environ 25°C, de manière à former un précipité, et à recueillir la liqueur surnageante contenant le fragment de complément.

5. Méthode suivant la revendication 4, dans laquelle le réactif est l'acrinol, le tampon est un tampon au phosphate 0,05M à pH 7,4 et le mélange est incubé pendant une durée allant de 20 minutes à une heure.

6. Méthode suivant l'une quelconque des revendications 1 à 5, dans laquelle le fragment de complément est le fragment $C_3a$ ou son dérivé des-Arg.

7. Méthode suivant l'une quelconque des revendications 1 à 5, dans laquelle le fragment de complément est le fragment $C_4a$ ou son dérivé des-Arg.

8. Méthode suivant l'une quelconque des revendications 1 à 5, dans laquelle le fragment de complément est le fragment $C_5a$ ou son dérivé des-Arg.

9. Kit sous une forme commerciale, dont les composants peuvent être assemblés en vue de leur utilisation dans l'analyse de liquides biologiques pour détecter un fragment de complément tel que défini dans la revendication 1, qui comprend

(a) un premier récipient renfermant une solution tamponnée de 0,8 à 1,6% d'un réactif tel que défini dans la revendication 1;

(b) un deuxième récipient renfermant le fragment de complément marqué; et

(c) un troisième récipient renfermant un anticorps qui reconnaît le fragment de complément.

10. Kit suivant la revendication 9, dans lequel le réactif est l'acrinol et qui comprend en outre

(a) un quatrième récipient renfermant un tampon d'analyse;

(b) un cinquième récipient renfermant un second anticorps;

(c) un sixième récipient renfermant le fragment de complément de référence à 25 ng;

(d) un septième récipient renfermant le fragment de complément de référence à 10 ng;

(e) un huitième récipient renfermant le fragment de complément de référence à 5,0 ng;

(f) un neuvième récipient renfermant le fragment de complément de référence à 2,5 ng; et

(g) un dixième récipient renfermant le fragment de complément de référence à 1,0 ng.